# EUROPEAN PATENT APPLICATION

(11) **EP 1 787 656 A1**
(43) Date of publication of application: **23.05.2007**
(21) Application number: 05773842.9
(22) Date of filing: 10.08.2005
(51) Int. Cl.: A61K 39/29, C12N 15/863, C07K 14/18

(54) **RECOMBINANT LIVE FOWLPOX VIRUS VECTORS AND THEIR USE IN PHARMACEUTICAL COMPOSITIONS AGAINST HEPATITIS C VIRUS**

(30) Priority: 11.08.2004 CU 1742004
(71) Applicant: CENTRO DE INGENIERIA GENETICA Y BIOTECNOLOGIA (CIGB), Ciudad de la Habana 10600 (CU)
(72) Inventor: ALVAREZ-LAJONCHERE PONCE DE LEÓN, Liz, San Rafael, Ciudad de La Habana 10400 (CU); DUEÑAS CARRERA, Santiago, San Rafael, Ciudad de La Habana 10400 (CU); GUERRA AIZPURUA, Ivis, Ciudad de La Habana 10600 (CU); ACOSTA RIVERO, Nelson, General Lee,20, altos, 10 de Octubre, Ciudad de La Habana 10500 (CU); MUSACCHIO LASA, Alexis, La Habana 32100 (CU)
(74) Representative: van Loon, C.J.J.
(86) International application number: PCT/CU2005/000004
(87) International publication number: WO 2006/015557

(57) **Abstract**

Recombinant fowl pox virus expressing hepatitis C virus proteins and their use in pharmaceuticals compositions, able to induce a cellular immune response against the hepatitis C virus, alter administration of a reduced number of doses. The pharmaceutical compositions of this invention are useful for prevention and treatment of infections associated with the hepatitis C virus.

SEQUENCE LISTING

<110> Centro de Ingeniería Genética y Biotecnología

<120> RECOMBINANT LIVE VECTORS AND THEIR USE IN PHARMACEUTICALS
COMPOSITIONS AGAINST HEPATITIS C VIRUS

<130> Seq VHC

<140>
<141>

<150> CU 2004-0174
<151> 2004-08-11

<160> 2

<170> PatentIn Ver. 2.1

<210> 1
<211> 831
<212> ADN
<213> DNA fragment derived from HCV coding region (nucleotide 237 to
1014)

<400> 1
atgaggcctc ccgggtaccc ttggcccctc tatggtaacg agggcatggg atgggcagga 60
tggctcctgt caccccgtgg ctctcggcct agttggggcc ccactgaccc ccggcgtagg 120
tcgcgtaatt tgggtaaggt catcgatacc ctcacatgcg gcttcgccga cctcatgggg 180
tacattccgc tcgtcggcgc ccccctaggg ggcgctgcca gggccctggc gcatggcgtc 240
cgggttctgg aggacggcgt gaattatgca acagggaatc tgcccggttg ctctttctct 300
ctcttccttt tggctttgct gtcctgtttg accatcccag tttccgccta tgaagtgcgc 360
aacgcgtccg gggtgtacca tgtcacgaac gactgctcca actcaagcat tgtgtatgag 420
gcagacgaca tgatcatgca cacccccgga tgcgtgccct gcgttcggga ggacaacacc 480
tcccgctgct gggtagcgct cacccccaca ctcgcggcca ggaatgccag cgtccccacc 540
acgacaatac gacgccacgt cgatttgctc gttggggcgg ctgctctctg ctccgctatg 600
tacgtggggg atctctgcgg atctgttttc ctcgtttccc agctgttcac cttctcgcct 660
cgccggcatg agacagcaca ggactgcaac tgctcaatct atcccggcca cgtatcaggt 720
caccgcatgg cctgggatat gatgatgaac tggtcacctt caacagccct agtggtatcg 780
cagttactcc ggatccgtcg acctgcagca tgccatggca taagtgacta g 831

<210> 2
<211> 602
<212> DNA
<213> Artificial sequence including HCV epitopes

<400> 2
atgggatcca cccacgtgac cggcggcagc caggcccgca ccacccacag cttcacctcc 60
ctgctgcgcc agggcgccaa gcagaacgtg cagctgatcg gcaaggtgat cgacaccctg 120

accagcggct tcgccgacct gatgggctac atcccactgg tgggcgcccc actgggcggc 180
gccgcccgcg ccaagaaggg ccacgtgagc ggccaccgca tggcctggga catgatgatg 240
aactgggcca gcaagaaggc cgccagccgc gccgccggct tgcaggacag caccatgctg 300
gtgagcgaag gccgcagctg ggcccagcct ggctacccat ggccactgta tggcaacgag 360
ggcatgggca agaagggctc cagcttcagc ctgttcctgt tggccctcct gagcagcttg 420
accatcaaga agatgagcta ctcctggacc ggcgccctgg tgaccccaag cgccgccgag 480
aagaagctgt tgttcaacat cctgggcggc tgggtgaaga agagcatggt gggcaactgg 540
gccaaggtga agaagtacac cggcgacttc gacagcgtga tcgactccag gccttaactg 600
ca 602

## Description

### Field of the invention

The present invention is related to the branch of immunology and viral vaccines, particularly with a recombinant fowl pox virus expressing proteins of hepatitis C virus (HCV) and pharmaceuticals compositions able to induce a cellular immune response against the HCV.

### Background previous art

The HCV is identified by first time in 1989, by using molecular biology techniques, as the main causal agent of non-A, non-B post-transfusional hepatitis (Choo Q-L., Kuo G., et al. (1989) Isolation of a cDNA clone derived from a blood-borne non-A, non-B viral hepatitis genome. Science. 244:359-62; EP0318216 Chiron Corp). Nowadays, more than 170 million people are infected with this viral agent worldwide. HCV infection is persistent in 85% of cases, frequently causing cirrhosis and hepatocellular carcinoma (Caselmann W.H., Alt M. (1996) Hepatitis C virus infection as a major risk factor for hepatocellular carcinoma. J. Hepatol. 24:61-66). In fact, after 25 years of infection, 25% of HCV chronic carriers develop cirrhosis and 1 to 4% of them develop hepatocellular carcinoma each year (Prince A.M., Shata M.T. (2001) Immunoprophylaxis of hepatitis C virus infection. Clin. Liver. Dis. 5:1091-103).

The treatment of choice for HCV infection is based on the combination of pegylated interferon administered in intensive regimens. However, this treatment is aggressive, expensive and effective in less than 50 % of cases. (Prince A.M., Shata M.T. (2001) Immunoprophylaxis of hepatitis C virus infection. Clin. Liver. Dis. 5:1091-103).

Different variants of protein subunits, virus like particles, synthetic peptides, recombinant live vectors and DNA constructs for DNA immunization, based on HCV antigens, have been evaluated as vaccination approaches (US6635257, US6387662, US6235888, US6685944, US2003021805, Lechmann M., Liang T.J. (2000) Vaccine development for hepatitis C. Semin. Liver. Dis. 20:211-26). Some of these variants have demonstrated their ability to elicit a specific immune response against HCV epitopes, although this response seems to be insufficient yet. At present, immunologic parameters correlating with protection against HCV infection have not been completely defined. Nevertheless, several studies suggest that an early, strong and multispecific cytotoxic T cell response, togheter with CD4+ Th1 cells, favors immunty against virus and resolution of disease. On the opposite, a late T cell response is related with the establishment of chronic liver immunopathology (Cerny A., Chisari F.V. (1999) Pathogenesis of chronic hepatitis C: immunological features of hepatic injury and viral persistente. Hepatology. 30:595-601; Cooper S., et al. (1999) Analysis of a successful immune response against hepatitis C virus. Immunity 10:439-49; Lechner F., et al. (2000) Why do cytotoxic T lymphocytes fail to eliminate hepatitis C virus? Lessons from studies using major histocompatibility complex class I peptide tetramers. Philos. Trans. R. Soc. Lond. B. Biol. Sci. 355:1085-92; Takaki A., et al. (2000). Cellular immune responses persist and humoral responses decrease two decades after recovery from a single-source outbreak of hepatitis C. Nat. Med. 6:578-82).

Recombinant live vectors are attractive candidates to generate a cellular immune response against HCV. Immunization in mice with adenovirus recombinant for HCV core and E1 elicited specific cytotoxic T cell response against these antigens (Bruna-Romero O., et al. (1997) Induction of cytotoxic T-cell response against hepatitis C virus structural antigens using a defective recombinant adenovirus. Hepatology 25:470-7). Although these results have been promising, recent problems with the use of recombinant adenovirus for gene therapy have generated doubts about their use in humans. The use of recombinant vaccinia and canary pox viruses, containing different HCV genes have induced cytotoxic T cell responses in mice, according the antigen evaluated, when they have been combined with vaccine candidates based on DNA constructs (Pancholi P., et al. (2000) DNA prime-canarypox boost with polycistronic hepatitis C virus (HCV) genes generates potent immune responses to HCV structural and nonstructural proteins. J. Infect. Dis. 182:18-27; Pancholi P., et al. (2003) DNA immunization with hepatitis C virus (HCV) polycistronic genes or immunization by HCV DNA priming-recombinant canarypox virus boosting induces immune responses and protection from recombinant HCV-vaccinia virus infection in HLA-A2.1-transgenic mice. J. Virol. 77:382-90). In fact, there is no evidence about the *in vivo* induction of cellular immunity after individual administration of canary pox viruses recombinant for HCV structural antigens, able to be effective, for instance, in a challenge model with recombinant vaccinia virus. To this group of viruses also belongs the fowl pox virus, which has not been used, up to now, for the induction of immune response against HCV antigens. The Fowl pox virus does not replicate in mammalian cells but can infect and express efficiently in the cytoplasm of the host cell the proteins codified by its genome. Due to these characteristics, the regulatory risks associated with the use of recombinant fowl pox in humans are reduced regarding to other live viral vectors.

Nowadays, there is no available vaccine against HCV infection in the market. Indeed, most studies are still in pre-clinical phase. Thus, the development of preventive and/or therapeutic agents effective against HCV infection is an unsolved problem and an urgent need.

### Desciption of the invention

The present invention, jumping over the limitations and the state of the art, solves the above mentioned problem at bringing a recombinant fowl pox virus containing DNA fragments derived from HCV, in a non-essential region of the fowl pox genome, able to induce a specific cellular immune response against HCV, according to the aa regions corresponding to HCV antigens expressed by the fowl pox virus.

According to the present invention, the virus FPCoE1 expresses a protein comprising aa 79-338 (SEQ ID No: 1), including a portion of the Core protein and E1, while the virus FPBS comprises a chimeric protein (SEQ ID No: 2), including epitopes specific for CD4+ and CD8+ T lymphocytes corresponding to different HCV antigens. In these viruses, the sequence coding for HCV antigens is derived from a cDNA of a HCV

Cuban isolate (Morales J, et al. WO 98/25960).

Another aspect of the present invention is referred to a pharmaceutical composition to induce a specific cellular immune response against the HCV, comprising an effective of a recombinant fowl pox virus and a pharmaceutically acceptable excipient.

The recombinant fowl pox viruses included in the present invention have the ability to penetrate the human cells and express the HCV antigens in the cytoplasm. The expression in human cells is controlled by the transcriptional unit based on an early/late synthetic promoter of fowl pox virus. These viruses also contain the xantin guanosin fosforribosil transferase gen under the control of 7.5K promote from vaccinia virus. These viruses are not able to replicate in humans.

This composition can be administered by intramuscular, intraperitonial or subcutaneous route. The method for administration can be by using syringes, gene gun, spray or other immunization devices. Each individual receives a dose ranging from 2.5 x 10⁷ to 1 x 10⁸ PFU/dose in a determined volume. In this invention, the procedures for administration of recombinant fowl pox viruses in prime/boost strategies with DNA constructs are also described. The recombinant fowl pox viruses can be also administered in prime/boost strategies with proteins or other viruses recombinant for HCV antigens or simultaneously with cytokines and other immunestimulatory molecules. These combinations allow the generation of new epitopic specificities or the enhancing of a specific branch of the immune response.

The pharmaceutical compositions based on fowl pox viruses recombinant for HCV antigens can have the following advantages:
- It is possible to induce a potent specific cellular immunity.
- A reduced number of immunizations is required to obtain the desired effect.
- The use of these compositions as the core for combined vaccines is possible.
- No adjuvant is required.
- They can be used in combination with antiviral agents.

According the circumstances, the pharmaceutical compositions can be administered to induce immunity against HCV before, simultaneously or after another therapy or vaccine. These pharmaceutical compositions can be administered to induce preventive immunity against HCV infection and also to induce immunity in patients with chronic hepatitis C, cirrhosis and liver cancer.

### Brief description of drawings

**Figure 1:** Schematic representation of the regions corresponding to HCV antigens in the recombinant fowl pox viruses.
**Figure 2:** Immunization schedule with the different recombinant fowl pox viruses. Antibody response (A). Interferon gamma secretory response (B). Response against the challenge with a vaccinia virus recombinant for HCV structural antigens (C).
**Figure 3:** Immunization schedule with FPCoE1 and FPBS by different routes of inoculation. Response against the challenge with the vaccinia virus recombinant for HCV structural antigens.
**Figure 4:** Immunization schedule with different amount of FPCoE1 and FPBS. Response against the challenge with a vaccinia virus recombinant for HCV structural antigens.
**Figure: 5:** Immunization schedule based on the combinations of FPCoE1 and a formulation of a DNA vaccine. Antibody response against Core, E1 and E2 (A). Response against the challenge with a vaccinia virus recombinant for HCV structural antigens (B).

### EXAMPLES OF REALIZATION

As is understood in the present invention, terms hepatitis C virus or HCV describe the virus in a generic manner, thus the terms are not limited to a particular viral sequence or HCV isolate.

Equally, sequence coding is understood as a nucleic acid molecule which is translated to a polypeptide, usually via mRNA, when is located under the control of an accurate regulatory sequence. In this case, coding sequence can include, but it is not limited to cDNA and recombinant nucleotide sequences.

The present invention will be described in a more detailed form by the following examples, which are illustrative and do not limit the goal of the invention.

### Example 1: Evaluation of FPCoE1c, FPCoE1 and FPBS

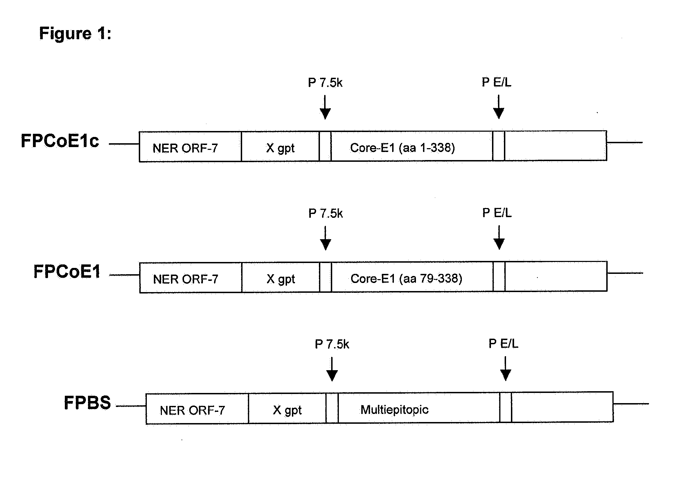

In order to evaluate the ability of fowl pox viruses recombinant for HCV antigens to induce a specific cellular immune response, the viruses FPCoE1c, FPCoE1 and FPBS were generated. The Figure 1 shows a schematic representation of the sequence corresponding to the HCV antigens. The viruses were obtained as follows: chicken embrio fibroblasts were infected with fowl pox and transfected with plasmids pFPCoE1c, pFPCoE1 or pFPBS by using lipofectamine (Invitrogen, USA). These plasmids are derived from plasmid pFP67xgpt (Vázquez-Blomquist D., González S., Duarte C.A. (2002) Effect of promoters on cellular immune response induced by recombinant fowlpox virus expressing multi-epitope polypeptides from HIV-1. Biotechnol. Appl. Biochem. 36:171-9*)* and contained the sequence coding for the regions comprising aa 1-338, aa 79-338 (SEQ ID No: 1), or the sequence coding for a chimeric protein based on epitopes specific for T lymphocytes (SEQ ID No: 2) (plasmids pFPCoE1c, pFPCoE1 and pFPBS, respectively). Later on, several cycles of viral purification in selective media with Xantine, hypoxantine and mycophenolic acid After that, the viral product from an infection plate was amplified and the presence of the insert corresponding to the HCV sequence was confirmed by polymerase chain reaction.

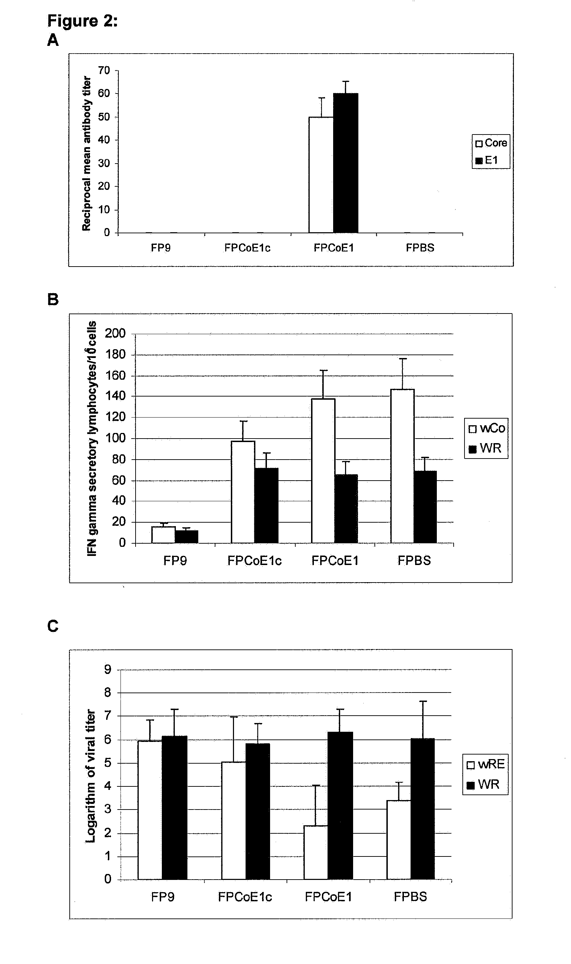

The immunogenicity of the viruses FPCoE1c, FPCoE1 and FPBS was studied in BALB/c female mice, 8 weeks old, after 2 intraperitoneal administrations (3 weeks apart) with 2.5x10⁷ PFU. Each immunization group included ten animals. Figure 2A shows that only the immunization with FPCoE1 was able to induce detectable levels of antibodies against Core and E1. However, Figure 2B shows an IFN-gamma secretion positive response from lymphocytes corresponding to animals immunized with FPCoE1 and FPBS, statistically significant with respect to the response detected in animals immunized with the negative virus, FP9 (Student T test, p<0.05). Immunization with FPCoE1c fail to induce a positive response of IFN-gamma secretion lymphocytes. Additionally, as shown in Figure 2C, animals from groups immunized with the viruses FPCoE1 and FPBS were able to significantly reduced (Student T test, p<0.05) the viral load in ovaries alter the challenge with 10⁶ PFU of a vaccinia virus (vvRE) recombinant for the HCV structural antigens (aa 1-650 of HCV polyprotein) with respect to the one observed after the challenge with the vaccinia virus control (WR), not recombinant for HCV antigens. Moreover, the vvRE viral load in animals immunized with the FPCoE1 and FPBS was statistically lower regarding the detected in animals immunized with the negative fowl pox virus FP9 (Student T test, p<0.05). Immunization with FPCoE1c did not significatively reduce the viral load in ovaries. The challenge with vaccinia virus was carried out by intraperitoneal inoculation, 2 weeks alter the last immunization with the fowl pox viruses. The extraction of ovaries was performed 5 days alter the inoculation of vaccinia virus. Challenge with recombinant vaccinia viruses has been widely used to demonstrate the ability of a vaccine candidate to elicit a strong cellular immune response *in vivo.* Particularly, the reduction of viral titer in ovaries is mainly related with the induction of strong CD8+ T lymphocyte response.

### Example 2: Routes of administration of fowl pox viruses (intraperitoneal, subcutaneous and intramuscular)

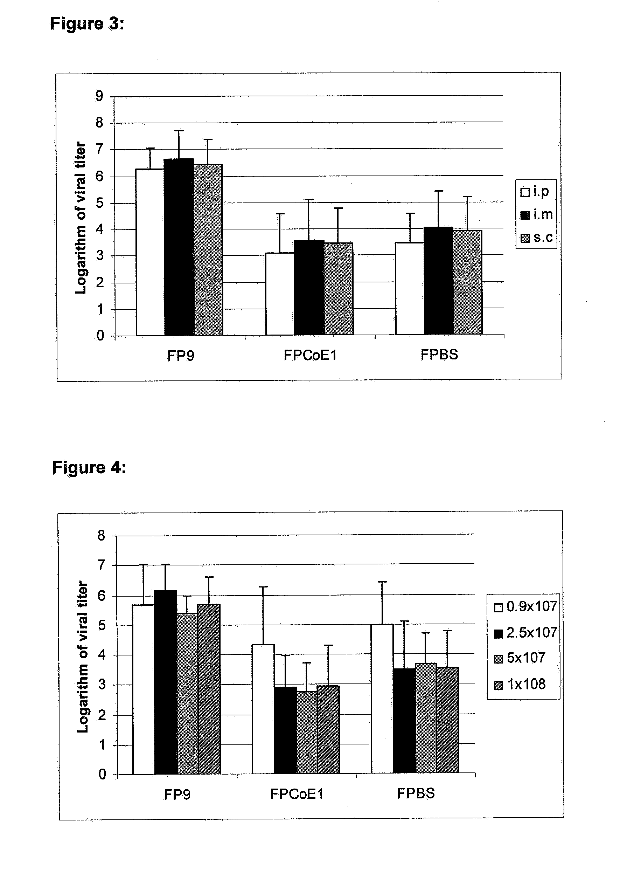

In order to evaluate the influence of the inoculation route on the immune response generated by the recombinant fowl pox viruses, female BALB/c mice, 8 weeks old, were immunized. Each immunization group included ten animals. Viruses (2.5x10⁷ PFU) were administered by intraperitoneal, intramuscular o subcutaneous route in two immunizations, 3 weeks apart. Figure 3 shows no significant difference among the immunization routes regarding the ability of recombinant fowl pox viruses FPCoE1 and FPBS to reduce viral load in ovaries after challenge with vaccinia virus vvRE. These 2 recombinant viruses induced a positive response based on the reduction of viral load with respect to the fowl pox negative control, FP9 (Student T test, p<0.05). The challenge with vaccinia virus was carried out by intraperitoneal inoculation of 10⁶ PFU of the vaccinia virus vvRE, 2 weeks alter the last immunization with the fowl pox viruses. The extraction of ovaries was performed 5 days alter the inoculation of vaccinia virus.

### Example 3: Doses comparison (0.9x10⁷, 2.5x10⁷, 5x10⁷ and 1x10⁸ PFU)

Female BALB/c mice were immunized by intramuscular route at weeks 0 and 3, with different amounts of the recombinant fowl pox viruses. Each immunization group included 10 animals. Figure 4 shows that mice immunized with 0.9x10⁷ PFU had no significant differences regarding the viral load detected in ovaries after challenge with 10⁶ PFU of vvRE, 2 weeks alter the last immunization with the fowl pox viruses. In contrast, the administration of 2.5x10⁷, 5x10⁷ and 1x10⁸ PFU of FPCoE1 and FPBS significantly reduced the vvRE viral load in ovaries with respect to the detected in animals inoculated with similar amounts of the fowl pox negative control FP9 (Student T test, p<0.05). In all cases, the extraction of ovaries was performed 5 days alter the inoculation of vvRE.

### Example 4: Prime/boost immunization with DNA and fowl pox viruses

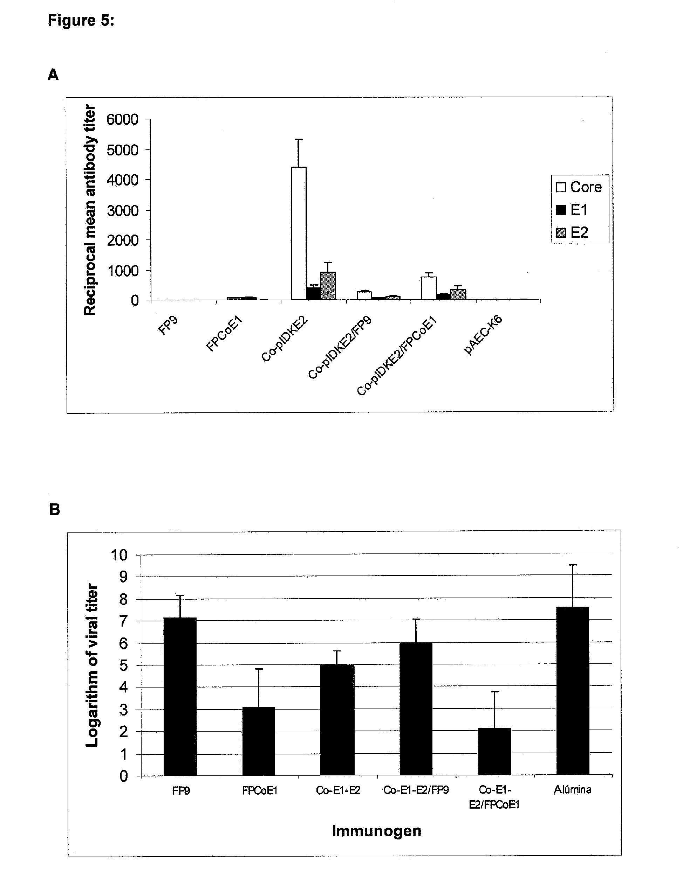

With the aim to evaluate the immune response induced in prime/boost immunizations combining a DNA vaccine candidate with a fowl pox virus recombinant for HCV antigens, female BALB/c mice, 8 weeks old, were immunized. Each immunization group included 10 animals. One group (Co-pIDKE2) was immunized by the intramuscular route at weeks 0, 3, 7 and 12 with a mixture of 100 µg of a plasmid expressing the first 650 aa of HCV polyprotein, pIDKE2 (Dueñas-Carrera S., et al. (2004). Immunization with a DNA vaccine encoding the hepatitis-C-virus structural antigens elicits a specific immune response against the capsid and envelope proteins in rabbits and Macaca irus (crab-eating macaque monkeys). Biotechnol Appl Biochem. 39:249-55) and 10 µg of a recombinant HCV core protein, Co.120 (Alvarez-Obregon J.C., et al. (2001). A truncated HCV core protein elicits a potent immune response with a strong participation of cellular immunity components in mice. Vaccine 19:3940-46). One group of animals (pAEC-K6) was immunized in similar conditions with a negative control plasmid pAEC-K6 (Herrera A.M., et al. (2000). A family of compact plasmid vectors for DNA immunization in humans. Biochem. Biophys. Res. Commun. 279:548-51). Another group was immunized with 2.5x10⁷ PFU of FPCoE1 by intramuscular route at weeks 0 and 3. Additionally, a group of animals was immunized with the negative fowl pox virus, FP9 in the same conditions. Moreover, one group (Co-pIDKE2/FPCoE1) was immunized by intramuscular route at weeks 0 and 3 with the mixture Co-pIDKE2 and in weeks 9 and 12 with FPCoE1. Another group (Co-pIDKE2/FP9) was immunized in similar way but the administrations at weeks 6 and 9 were administered with FP9. Figure 5 shows that the highest levels of antibodies were induced in the group Co-pIDKE2 (Student T test, p<0.05). Moreover, the prime/boost administration, combining Co-pIDKE2 and FPCoE1 induced higher levels of antibodies regarding those detected in the group immunized with FPCoE1 alone (Student T test, p<0.05). In Figure 5 B, it is possible to observe that both groups immunized with FPCoE1, or Co-plDKE2, as well as the group immunized with Co-plDKE2 in the first 2 doses and FPCoE1 in the other 2, significantly reduced (Student T test, p<0.05) the vvRE viral load after challenge with respect to the animals immunized with the negative controls. The lowest viral load was detected in the animals immunized with Co-pIDKE2 in the first 2 doses and FPCoE1 in the other 2. The challenge with vaccinia virus was carried out by intraperitoneal inoculation of 10⁶ PFU of the vaccinia virus vvRE, 2 weeks alter the last immunization. The extraction of ovaries was performed 5 days alter the inoculation of vaccinia virus vvRE.

## Claims

1. A recombinant fowl pox virus containing DNA fragments derived from HCV, in a non-essential region of the fowl pox virus genome, able to induce a specific cellular immune response against HCV antigens.

2. A fowl pox virus according to claim 1, where the DNA fragment derived from HCV is the sequence coding for a polyprotein Core-E1 comprising aa 79-338 (SEQ ID No: 1).

3. A fowl pox virus according to claim 1, where the DNA fragment derived from HCV is the sequence coding for a chimeric protein base don epitopes specific for T lymphocytes (SEQ ID No: 2).

4. A fowl pox virus according to claims 1 and 2, which is the FPCoE1.

5. A fowl pox virus according to claims 1 and 3, which is the FPBS.

6. A pharmaceutical composition to induce a specific cellular immunity against HCV, comprising an effective immunizing amount of the recombinant fowl pox virus according to claims 1 to 5 and a pharmaceutically acceptable excipient.

7. A pharmaceutical composition according to claim 6, where the effective immunizing dose ranges from 2.5x10⁷ to 1x10⁸ PFU/dose.

8. A pharmaceutical composition according to claims 6 and 7 to induce preventive immunity against HCV infection.

9. A pharmaceutical composition according to claims 6 and 7 to induce immunity against HCV in patients with chronic hepatitis C, cirrhosis and liver cancer.

10. A pharmaceutical composition according to claims 6 and 7 to induce immunity against HCV before, simultaneously or after other therapy or vaccine.
